# EUROPEAN PATENT SPECIFICATION

(11) **EP 1 005 844 B1**
(45) Date of publication and mention of the grant of the patent: **19.03.2003**
(21) Application number: 97934818.2
(22) Date of filing: 28.07.1997
(51) Int. Cl.: A61F 13/00, A61L 15/16, A61L 15/32, A61L 15/44

(54) **BANDAGE CLOTH HAVING AN EXTENDED THERAPEUTIC ACTION**
VERBANDSGEWEBE MIT VERLÄNGERTER THERAPEUTISCHER WIRKUNG
TISSU POUR BANDAGES AYANT UNE ACTION THERAPEUTIQUE PROLONGEE

(43) Date of publication of application: 07.06.2000
(73) Proprietor: Vasilieva, Tatiyana Sergeevna, Moscow, 117313 (RU); Subbotko, Olga Alexandrovna, Moscovskaya obl., 140192 (RU)
(72) Inventor: Vasilieva, Tatiyana Sergeevna, Moscow, 117313 (RU); Subbotko, Olga Alexandrovna, Moscovskaya obl., 140192 (RU)
(74) Representative: Herden, Andreas F.
(86) International application number: RU9700241
(87) International publication number: WO99004738

(56) References cited:
- DE-A- 4 445 003
- RU-C- 2 031 661
- US-A- 4 009 313
- US-A- 4 837 024
- US-A- 5 035 893
- 3 Z.F. VASILKOVA et al., "Plenochnye i Gubchatye Polifunktsionalnye Perevyazochnye Pokrytya na Polimernoi Osnove v Lechenu Gnoinykh Ran", I INTERNATIONAL CONFERENCE MODERN APPROACHES TO THE DEVELOPMENT OF THE EFFECTIVE DRESSING MATERIALS AND POLYMER IMPLANTANTS, 25-26 November 1992, Moscow, p. 152-153.

## Description

### Technical Field

The present invention relates in general to medicine and has particular reference to dressing material producing a prolonged therapeutic effect.

### Background Art

Known from prior art is a dressing (US Patent # 4,727,868 A) producing no therapeutic effect, wherein used as a covering material for wounds is knitted web foulard-treated with film-forming polyurethane solution. However, despite good atraumatic characteristics the material in question features too a low adsorptive capacity with respect to exudate, and is poorly wettable therewith.

Known in the present state of the art is a medical-purpose textile structure producing no therapeutic effect (cf. US Patent # 4,542,739 A), wherein use is made of a warp knitted fabric having the weft made from highly adsorptive fibers, such as cotton, flax, viscous-rayon, whereas a netting or looping texture facing the wound surface is formed from synthetic fibres (polyamide, polyester, polyurethane). Such webs though having good atraumatic characteristics, feature but inadequate wetting rate and moisture absorbing capacity, which narrows substantially the field of their application, restricting it solely to closure of dry wounds or of wounds with a small amount of discharge. Furthermore, textures of warp-knitted webs has but low pliability, which makes it impossible to model the surface of intricately shaped wounds or form drains or turundae, thereby still more restricting field of application of dressings from warp-knitted webs for covering wounds and burns.

One prior-art prolonged-action dressing material is known to comprise a base material or carrier having an essentially pliable or movable texture made, e.g., of a fibrous material, paper, or film) an medicated with a complex of a medicinal agent and biocompatible polymer applied to at least on one side of the material, said complex comprising 10 to 40 parts by weight of a liquid organic polyoxy compound, i.e., glycerol, ethylene glycol, or propylene glycol, and 90 to 60 parts by weight of a gel-forming water-insoluble polymer possessing high adsorbability (up to 10 g/g), i.e., polyacrylates or modified polysaccharides (such as carboxymethyl cellulose) (EP # 0096976 B1, IPC A 61 L 15/00).

The dressing material under discussion is adapted for active sorption of body fluids (blood, urine, and the like), as being in fact an adsorbent which can appear as a film or drape used in wound treating, as well as squares or hygienic pledgets and stypes for female patients.

However, said material cannot be used for modeling intricately shaped wounds and burnt spots because the texture of the carrier used in said material is devoid of the necessary pliability and plasticity.

### Disclosure of the Invention

The present invention has for its principal object to provide, due to appropriate structural and arrangement modifications, a dressing material capable of producing a prolonged therapeutic effect and having improved ability to model the surface of the affected areas to be protected.

The foregoing object is accomplished due to the fact that in a dressing material producing a prolonged therapeutic effect, comprising a carrier coated on both sides thereof with a complex of a biocompatible polymer and a medicinal agent, according to the present invention, said dressing material comprises a circular knitted texture from cotton fibres as the pliable-texture carrier, and a biodegradable polymer as the biocompatible polymer, the amount of said deposited polymer and said medicinal agent equalling from 15 to 70 g/sq.m and said components being taken in equal parts, on both sides of the carrier.

The proposed dressing material is capable of producing a prolonged therapeutic effect due to its having high moisture absorptive capacity and wettability. In addition, the proposed dressing material features an enhanced ability to model wound surfaces.

According to the invention, it is expedient that the circular knitted carrier be made of cotton fibres in a mixture with viscose-rayon fiber, the weight ratio between cotton fibres and viscose-rayon fiber being from 80:20 to 50:50, respectively.

According to the invention, it is reasonable that one or two polysaccharides be used as the biocompatible polymer.

It is also desirable that the proposed dressing material comprise, as the biocompatible polymer, a mixture of a polysaccharide and collagen taken in a weight ratio of from 80:20 to 50:50, or else a mixture of a polysaccharide and gelatin taken in a weight ratio of from 80:20 to 50:50.

According to the invention, it is desirable that the herein- proposed dressing material be provided, on at least one side thereof, with a coating made from a fibrous material, either woven or nonwoven, or, more specifically, from a nonwoven plain-knitted cloth or a polyester net.

Further objects and advantages of the present invention will become apparent from the detailed description of the dressing material producing a prolonged therapeutic effect, and specific exemplary embodiments thereof.

### Best Method of Carrying Out the Invention

The herein-proposed dressing material is capable of producing a prolonged therapeutic effect, has improved hygienic properties, in particular, moisture absorbing capacity and wettability, as well as better ability to model wound surfaces.

Furthermore, more pliable and elastic texture of the dressing material and the aforementioned type of fiber of the textile web thereof provide for the ability of the material to readily model the surface of wounds, especially of wounds and burns located in hard-of-access places, e.g., wounds following the opening of abscesses and phlegmons or burns of the hands, and so on.

The proposed dressing material features improved consumers' properties and is convenient in use because it is suitable for covering of wounds and injured tissues with either of its sides which allows of, firstly, utilizing the material quickly for rendering first medical aid, especially under extreme conditions (e.g., mass disasters, emergency situations, etc.) and secondly, using it as, e.g., turundae, drains, and so on. The material is colored differently which also adds to its consumers' properties by facilitating identification of the material; provision of the complex of a medicinal agent and a polymer on both sides of web in equal parts also contributes to better consumers' properties of the proposed material.

The material is atraumatic as being not causative pain sensations when applying and changing dressings.

Furthermore, a possibility of inserting extra layers of any materials made use of in medicine, into the proposed dressing material allows of further improvements in its sorptive and atraumatic properties and also of using the material, in particular, for treating wounds featuring profuse secreta.

The herein-proposed dressing material producing a prolonged therapeutic effect is available in diversified shapes, e.g., napkins, turundae, pledgets, plasters, and so on.

Depending on its purpose the proposed material may be colored differently.

When in the form of a circular knitted textile web the proposed dressing material may comprise any cotton fiber or its mixture with viscose-rayon fiber, the weave structure of said fibers may be any (that is, ribbed 1x1, hosiery, interlock, chaminite, and others).

With the cotton-fiber-to-viscose-rayon fiber ratio in the web lying within 80:20 and 50;50, the cost of the material is reduced while its hygienic properties are enhanced (up to 20%).

The proposed dressing material may comprise any one of suitable polymers as the biocompatible biodegradable polymer, including one or more polysaccharides, as well as mixtures thereof with, e.g., collagen and gelatin.

The polysaccharide-to collagen or polysaccharide-to gelatin ratio of the mixture being from 80:20 to 50:50, respectively, allows of still more improving the moisture-absorbing and therapeutic properties of the dressing material.

Use of at least two polysaccharides as a biocompatible biodegradable polymer adds to the therapeutic properties of the proposed dressing material by, e.g., imparting hemostatic properties thereto.

The proposed dressing material may comprise as a medicinal substance at least one of the drugs having an antipyretic, antiseptic, hemostatic, or other therapeutic effect, as well as mixtures thereof and some other medicinal preparations.

The proposed material of each specific application has color of its own, thus facilitating its identification.

A medicinal substance with which the proposed dressing material is medicated, may comprise, apart from the drug producing the principal target-oriented effect, also the components producing an additional therapeutic effect, e.g., medical-use oils, vitamins, infusions of medicinal plants, dyes and stains permitted for use in medicine, thus extending the field of application of the proposed dressing material.

The complex with which the herein-proposed dressing material is medicated, comprising a biocompatible biodegradable polymer and a medicinal substance, is applied thereto in an amount of from 15 to 70 g/sq.m in equal parts to both sides of the web.

When the amount of the complex is below 15 g/sq.m, the proposed dressing material fails to produce an adequately prolonged therapeutic effect, and when the amount of the complex exceeds 70 g/sq.m, the complex is liable to flake off from the web surface during use of the material.

The fact that the therapeutic complex is applied on both web sides in equal amounts allows the material to be used quickly for treating the surface of a wound or burn, especially in the case of its being intricately shaped.

The proposed dressing material can further comprise at least one layer which is made from any materials permitted for use in medicine and is aimed at improving the sorption and/or atraumatic properties of said dressing material.

The proposed dressing material is produced using a routine production process and standard equipment and is utilized for therapeutic purposes (treating wounds, etc.) following commonly adopted procedures.

Therapeutical properties of the proposed dressing material were trialed clinically on patients exhibiting surgical and other pathologies.

For instance, the proposed dressing material in the form of dressings measuring 10x7.5 cm was used at the Chair of general surgery of the Moscow Sechenov Medical Academy for treating a total of 50 patients with surgical pathologies of different etiology and localization:

| | |
|---|---|
| purulent wounds | -6 |
| infected wounds | -4 |
| phlegmons, abscesses | -8 |
| trophic ulcers | - 5 |
| osteomyelitis (fistulas) | 5 (pre- and postoperative) |
| mastitis | -1 |
| purulent bursitis | -1 |
| acute appendicitis | -6 |
| celotomy | -5 (including two strangulated) |
| gastrectomy | -4 |
| phlebectomy | -2 |
| lipoma | -1 |
| laparotomy | -1 |
| Total | 50 patients. |

There were 33 male and 17 female patients. The age bracket of the patients was 19 to 83.

The material was used at the first and second stages of the wound healing process when treating patients with purulent wounds and trophic ulcers, as well as in the postoperative period for treating primary sutures resulting from preplanned and emergency surgeries.

Preliminary toilet of the wound with a 1:5000 furacilin solution, a 3% hydrogen peroxide solution, chloramine, and chlorhexidine bigluconate was followed by applying a bandage from the proposed dressing material to the surface of a wound or trophic ulcer; in case of a deeply seated operative wound or a fistula, a turunda was formed from a drape, with the aid of which draining of the wound was effected. Bandages were changed daily, and within the postoperative period in the case of a blind suture they were changed every other or two days.

The utilization efficiency of the dressing material was assessed on the grounds of clinical observations, bacterial and cytological examinations. The following characteristics were determined during the aforesaid examinations: imbibing capacity of drapes, specific microbial sorption, wetting rate, pliability and plasticity, and strength of a dressing before and after its use. The following results were obtained:
- the dressings were found to have good ability to imbibe wound secreta, to produce a pronounced sorptive effect, to perform a good draining function, to reduce the degree of bacterial contamination of wounds and trophic ulcers due to the effect of both sorption and an antiseptic;
- an analysis into the microflora composition demonstrated that a majority of patients (66%) exhibited as pathogens Staphylococcus aureus and Staphylococcus hemolyticus appearing as a monoculture and as an association with other microorganisms (in 18% of the patients).

It has been established during the examination that use of dressings made from the proposed dressing material yields the following positive results:
- the process of healing wounds and trophic ulcers of the various localization and etiology is accelerated;
- edema and hyperemia of wound lips and suture region are abated;
- number of redressings is reduced;
- the dressings are convenient in use, they follow readily the wound shape and the ulcer surface, their packaging and size are convenient as allowing of determined individually the number of pieces with account of the area and configuration of the wound surface;
- good results are yielded in treating burns and incised wounds;
- good results are also obtained from the use of this a dressing material applied to the postoperative aceptic sutured wounds, whereby the number of pyogenic complications and postoperative suppurations is reduced by 40 to 50%;
- the dressings protect clean granulating wounds and suture region against the effect of the surrounding atmosphere, thus preventing the wounds from reinfection.

In addition, the proposed dressing material comprising carboxymethyl cellulose and medicated with the anticeptic furagin and with chlorhexidine bigluconate, respectively, were clinically studied at the Vishnevsky Institute of Surgery.

The dressings were used in 15 patients treated for wounds with various-genesis purulent inflammations (that is, abscesses, combustial wounds, various-etiology trophic ulcers, purulent postoperative wounds). Studies were performed in a comparative aspect with routine treatment methods.

The course of the wound-healing process was monitored against the clinical data available (i.e., arresting the inflammatory process, the appearing of granulations, vascularization, and an epithelial layer), the duration of the healing period, and with the aid of dynamic microbiological examination.

The wounds were bound up daily.

According to observation evidence, any inflammatory symptoms virtually disappeared on the second or third day after dressing adjustment. Wound discharge was reduced and became of the serose character. Points of nascence of granulations were found to appear. On the fifth or seventh day of treatment the wounds were found to get clean and covered with bright succulent granulations.

A progress in wound healing was observed throughout the period of use of the proposed dressing material. On the ninth day the wound healing process got clearly defined, i.e., fine granulations were found to appear and every wound developed marginal epithelization.

All the wounds treated (less trophic ulcers) were found to completely heal on the 12th-14th day of treatment.

According to evidence of quantitative microbiological analysis, the pretreatment wounds exhibited high degree of microbial contamination (above the critical level). Dynamic observation demonstrated that more pronounced reduction in the amount of microbes resulted from use of a dressing medicated with chlorhexidine bigluconate. On the third or fifth day the microflora level in the wounds was found to be approximately twice as low as before treatment. This allowed of closing the wounds using diverse techniques (e.g., by suturing, using autodermatoplasty or cell culture). By the ninth day of treatment as low as 10 to 100 microflora colonies were taken from the wounds which did not prevent their epithelization and healing.

Clinical studies of the dressings demonstrated them to be applicable for treating wounds with pronounced purulent inflammation of the various genesis (that is, I-III degree combustial wounds, suppurative wounds, wounds after primary surgical treatment, and heavily exudative trophic ulcers.

Similar trials were also conducted at the Chair of general surgery of the Russian State Medical University.

Subjected to trialing were 250 sterile antimicrobial dressings measuring 10x7 cm medicated with furagin and another 250 similar dressings medicated with chlorhexidine bigluconate, enclosed in high-pressure polyethylene package.

The dressings were applied to primary sutures used within the postoperative period following preplanned and emergency surgeries, as well as for treating patients with purulent wounds and trophic ulcers at the first and second stages of the wound healing process.

Drapes from the proposed dressing material wetted with a furacilin or hydrogenperoxide solution, or else physiological saline were applied to the surface of a wound or ulcer. Whenever a deeply seated postoperative wound or fistulas were involved, turundae were formed from the dressing for better wound draining. As the wound was cleaned, edema and exudation subsided, redressing rate became lower (every other day or two days). There was clinically observed complete cleaning of the wounds within an average period of four to ten days. Alleviation of pain, abatement of edema and hyperemia, and an improved general state of the patients were noted.

The utilization efficiency of the dressing material was assessed on the grounds of clinical observations. The dressings were found to imbibe wound exudate well and to possess pronounced draining properties. They were also found to be convenient in use and to readily follow the shape of a wound, and their packaging and size were found to allow of determining individually the number of pieces with account of the area and configuration of the wound surface.

Use of proposed dressing material is found to:
- accelerate the processes of cleaning and healing of wounds and trophic ulcers;
- abate edema and hyperemia of the lips and suture region of wounds;
- reduce the rate of incidence of purulent complications when the dressing material applied to the surface of postoperative aseptic sutured wounds;
- protect clean granulating wounds and suture region against the effect of the surrounding atmosphere, thus preventing the wounds from reinfection.

To promote understanding of the present invention and with a view to its better illustration, tabulated below are some specific exemplary embodiments of the proposed material which place no limitation upon the scope of the present invention, and the results of trials of said material for hygienic and other properties, as well as for therapeutic effect produced thereby.

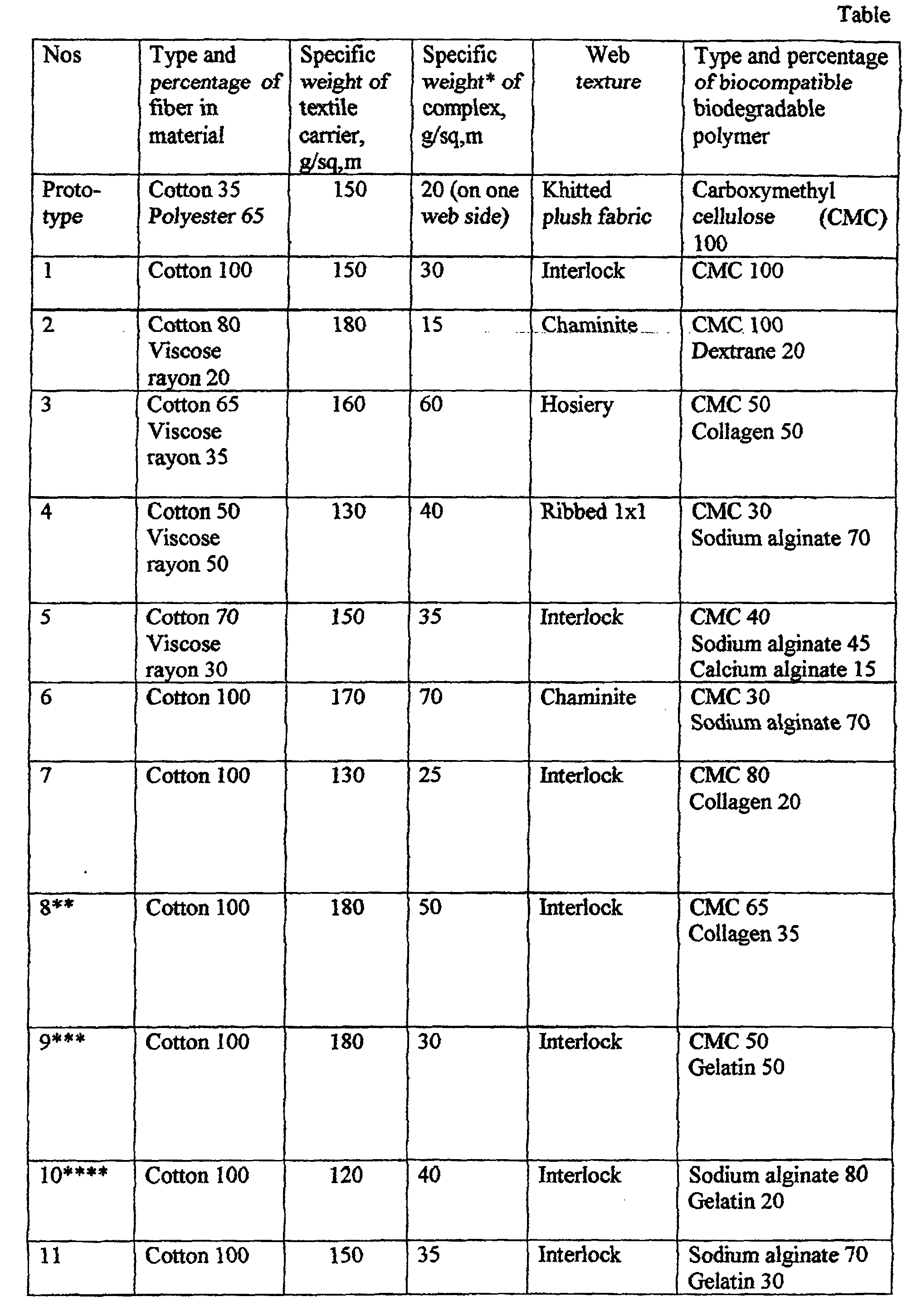

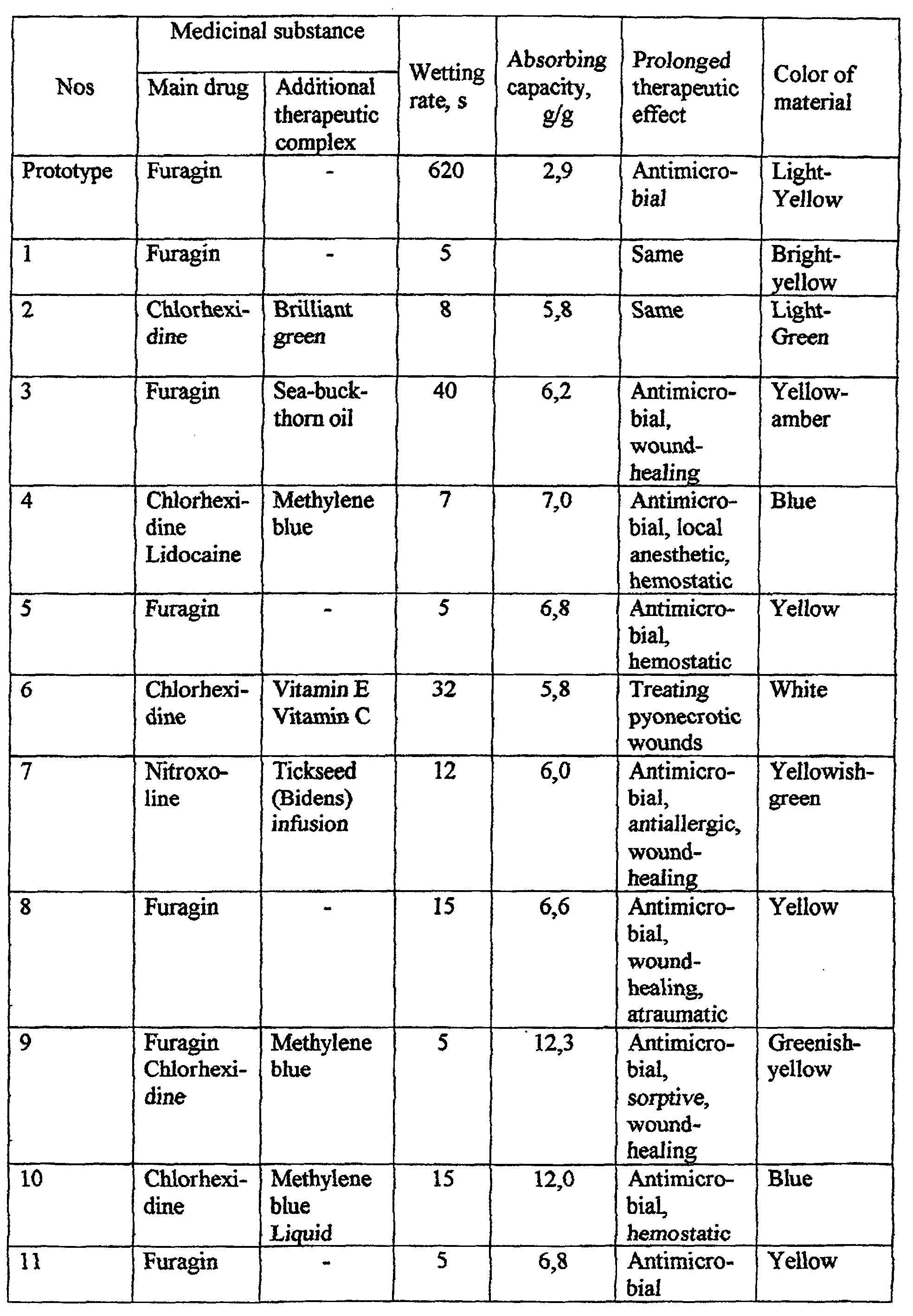

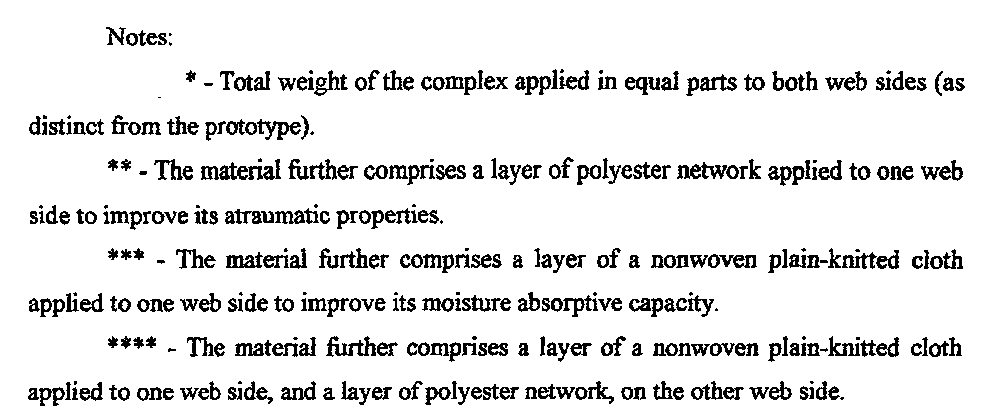

### Industrial Applicability

The proposed dressing material can be used in surgery for treating burns and wounds of the various etiology, for closing injured tissues, treating pustular and trophic lesions of skin and subcutaneous fat.

## Claims

1. A dressing material capable of producing a prolonged therapeutic effect, comprising a carrier coated on both sides thereof with a complex of a biocompatible polymer and a medicinal agent, according to the present invention, said dressing material comprises, as the pliable-texture carrier, a circular web from cotton fibres, and as the biocompatible polymer, a biodegradable polymer, the amount of said deposited polymer and said medicinal agent equals from 15 to 70 g/sq.m and said components are taken in equal parts on both sides of the carrier.

2. The dressing material of claim 1, **CHARACTERIZED in that** the circular knitted texture is made of cotton fibres in a mixture with viscose-rayon fiber, the weight ratio between cotton fibres and viscose-rayon fiber being from 80:20 to 50:50, respectively.

3. The dressing material of claim 1, **CHARACTERIZED in that** it comprises a polysaccharide as the biocompatible polymer.

4. The dressing material of claims 1 are 2, **CHARACTERIZED in that** it comprises two polysaccharides as the biocompatible polymer.

5. The dressing material of claim 1 are 2, **CHARACTERIZED in that** it comprises, as the biocompatible polymer, a mixture of a polysaccharide and collagen taken in a weight ratio of from 80:20 to 50:50.

6. The dressing material of claim I and 2, **CHARACTERIZED in that** it comprises, as the biocompatible polymer, a mixture of a polysaccharide and gelatin taken in a weight ratio of from 80:20 to 50:50.

7. The dressing material of claim 1 to 6, **CHARACTERIZED in that** it comprises on at least one side thereof, a coating made from a fibrous material, either woven or nonwoven or, more specifically, from a nonwoven plain-nitted cloth or a polyester network.

8. The dressing material of claim 7, **CHARACTERIZED in that** said coating is made from a nonwoven plain-nitted cloth.

9. The dressing material of claim 7, **CHARACTERIZED in that** said coating is made from a polyester network.

## Patentansprüche

1. Verbandsmaterial, welches eine verlängerte therapeutische Wirkung hervorzubringen vermag, das einen Träger umfasst, der entsprechend der vorliegenden Erfindung auf seinen beiden Seiten mit einem Komplex aus einem biologisch verträglichen Polymer und einem Heilmittel beschichtet ist, wobei das Verbandsmaterial als den Träger mit biegsamer Textur ein kreisförmiges Gewebe aus Baumwollfasern und als das biologisch verträgliche Polymer ein biologisch abbaubares Polymer umfasst, wobei die Menge des aufgebrachten Polymers und des Heilmittels 15 bis 70 g/m² beträgt und die Bestandteile auf beiden Seiten des Trägers zu gleichen Teilen genommen werden.

2. Verbandsmaterial nach Anspruch 1,
**dadurch gekennzeichnet, dass** die kreisförmige, gewirkte Textur aus Baumwollfasern in einer Mischung mit Viskosereyonfasern hergestellt ist, wobei das Gewichtsverhältnis zwischen Baumwollfasern und Viskosereyonfasern jeweils von 80:20 bis 50:50 beträgt.

3. Verbandsmaterial nach Anspruch 1,
**dadurch gekennzeichnet, dass** es als das biologisch verträgliche Polymer ein Polysaccharid umfasst.

4. Verbandsmaterial nach Anspruch 1 oder 2,
**dadurch gekennzeichnet, dass** es als das biologisch verträgliche Polymer zwei Polysaccharide umfasst.

5. Verbandsmaterial nach Anspruch 1 oder 2,
**dadurch gekennzeichnet, dass** es als das biologisch verträgliche Polymer eine Mischung aus einem Polysaccharid und Kollagen, in einem Gewichtsverhältnis von 80:20 bis 50:50 genommen, umfasst.

6. Verbandsmaterial nach Anspruch 1 und 2,
**dadurch gekennzeichnet, dass** es als das biologisch verträgliche Polymer eine Mischung aus einem Polysaccharid und Gelatine, in einem Gewichtsverhältnis von 80:20 bis 50:50 genommen, umfasst.

7. Verbandsmaterial nach Anspruch 1 bis 6,
**dadurch gekennzeichnet, dass** es zumindest auf einer seiner Seiten eine Beschichtung umfasst, die aus Fasermaterial hergestellt ist, das entweder gewebt oder nicht gewebt ist oder, spezieller, aus einem nicht gewebten, flachgestrickten Stoff oder einem Polyesternetz hergestellt ist.

8. Verbandsmaterial nach Anspruch 7,
**dadurch gekennzeichnet, dass** die Beschichtung aus einem nicht gewebten, flachgestrickten Stoff hergestellt ist.

9. Verbandsmaterial nach Anspruch 7, **dadurch gekennzeichnet, dass** die Beschichtung aus einem Polyesternetz hergestellt ist.

## Revendications

1. Matière de pansement capable de produire un effet thérapeutique prolongé, comprenant un support revêtu sur ses deux faces avec un complexe d'un polymère biocompatible et d'un agent médicamenteux, conforme à la présente invention, ladite matière de pansement comprenant, comme support à texture souple, une bande circulaire formée de fibres de coton et, comme polymère biocompatible, un polymère biodégradable, la quantité dudit polymère déposé et dudit agent médicamenteux étant comprise dans l'intervalle de 15 à 70 g/m² et lesdits constituants étant présents en parties égales sur les deux faces du support.

2. Matière de pansement suivant la revendication 1, **caractérisée en ce que** la texture tricotée circulaire est constituée de fibres de coton en mélange avec des fibres de viscose-rayonne, le rapport pondéral entre les fibres de coton et les fibres de viscose-rayonne, respectivement, étant compris dans l'intervalle de 80:20 à 50:50.

3. Matière de pansement suivant la revendication 1, **caractérisée en ce qu'**elle comprend un polysaccharide comme polymère biocompatible.

4. Matière de pansement suivant les revendications 1 et 2, **caractérisée en ce qu'**elle comprend deux polysaccharides comme polymère biocompatible.

5. Matière de pansement suivant les revendications 1 et 2, **caractérisée en ce qu'**elle comprend, comme polymère biocompatible, un mélange d'un polysaccharide et de collagène présents en un rapport pondéral compris dans l'intervalle de 80:20 à 50:50.

6. Matière de pansement suivant les revendications 1 et 2, **caractérisée en ce qu'**elle comprend, comme polymère biocompatible, un mélange d'un polysaccharide et de gélatine présents en un rapport pondéral compris dans l'intervalle de 80:20 à 50:50.

7. Matière de pansement suivant les revendications 1 à 6, **caractérisée en ce qu'**elle comprend, sur au moins une de ses faces, un revêtement constitué d'une matière fibreuse, tissé ou non tissé ou bien, plus spécifiquement, d'une étoffe non tissée à tricot uni ou d'un réseau de polyester.

8. Matière de pansement suivant la revendication 7, **caractérisé en ce que** ledit revêtement est constitué d'une étoffe non tissée à tricot uni.

9. Matière de pansement suivant la revendication 7, **caractérisée en ce que** ledit revêtement est constitué d'un réseau de polyester.
